# EUROPEAN PATENT APPLICATION

(11) **EP 4 660 905 A1**
(43) Date of publication of application: **10.12.2025**
(21) Application number: 25181584.1
(22) Date of filing: 09.06.2025
(51) Int. Cl.: G06Q 10/00, G01W 1/00

(54) **SYSTEMS AND METHODS FOR AN ATMOSPHERIC AND ASSET-BASED MANAGEMENT AND CONTROL PLATFORM**

(30) Priority: 07.06.2024 US 202418736972
(71) Applicant: Scepter Incorporated, San Francisco, California 94115 (US)
(72) Inventor: FATHER, Philip Raymond, SAN FRANCISCO, 94115 (US)
(74) Representative: Boult Wade Tennant LLP

(57) **Abstract**

Disclosed are systems and methods that provide a decision intelligence (DI)-based computerized framework for deterministically determining atmospheric data and patterns in/around locations, and leveraging such information for on-demand access and usage on similar sites. The disclosed systems and methods can provide a synergistic combination of a layered data repository, DI-based computational analysis, sensor integration and simulation capabilities, which can provide an interactive holistic system for monitoring, analyzing and managing atmospheric conditions at specific locations. The disclosed framework can provide unprecedented insights, enabling systems, sites, assets and/or users to make informed decisions, optimize asset operations and proactively mitigate environmental impacts.

## Description

### FIELD OF THE DISCLOSURE

The present disclosure is generally related to an electronic climate management system, and more particularly, to a decision intelligence (DI)-based computerized framework for deterministically determining atmospheric data and patterns in/around locations, and leveraging such information for on-demand access and usage on similar sites.

### SUMMARY OF THE DISCLOSURE

The disclosed systems and methods provide a pioneering computerized framework that provide novel mechanisms for monitoring and managing atmospheric data, inclusive in how it can be accessed and leveraged for other locations and/or assets. As discussed herein, the disclosed framework provides a comprehensive, data-driven solution for climate monitoring (e.g., methane and air pollution, for example), paving the way for proactive environmental stewardship and global collaboration.

Accordingly, as discussed herein, disclosed herein are systems and methods for a comprehensive atmospheric monitoring and environmental management framework. In some embodiments, the framework can include, and/or have access to a sensor network integrated into and/or associated with vertical air columns around terrestrial assets for collecting atmospheric data. The framework can further be associated with a database (e.g., a distributed ledger technology, for example) that can securely store and layer the atmospheric data with asset data, meteorological data, and/or other relevant datasets, which can enable secure access and provide audit trails for determined actions and/or outputs.

Moreover, in some embodiments, the disclosed framework can perform DI operations via artificial intelligence and/or machine learning (AI/ML) techniques, such that the layered data stored in the database can be subject to AI/ML computational analysis in order for the development of predictive models of atmospheric conditions and environmental impacts. The framework can then optimize terrestrial sensor configurations and operational systems based on the results of the AI/ML analysis, such that the configuration and/or operations of such terrestrial sensor configurations can operate in a manner that is non-native to the preset configurations.

In some embodiments, the framework can generate virtual representations (or simulations) of vertical air column conditions for scenario testing and strategic planning.

Accordingly, as discussed herein, the integrated framework can provide functionality that enables proactive environmental stewardship through continuous monitoring, data-driven insights, cross-asset optimization, and simulation-based decision support. The framework facilitates sustainable operations, regulatory compliance, and collaborative environmental efforts across industries and geographical borders. Indeed, by leveraging cutting-edge technologies, such as, for example, sensor networks, data analytics, cloud computing, blockchain, AI/ML, and the like, or some combination thereof, the disclosed framework can redefine the way operations for monitoring and managing atmospheric conditions are performed. The framework can provide actionable insights, enable proactive environmental management, and foster cross-border and cross-industry collaboration, paving the way for a sustainable future where clean skies are not merely a goal but a sustained reality.

According to some embodiments, a method is disclosed for a DI-based computerized framework for deterministically determining atmospheric data and patterns in/around locations, and leveraging such information for on-demand access and usage on similar sites. In accordance with some embodiments, the present disclosure provides a non-transitory computer-readable storage medium for carrying out the above-mentioned technical steps of the framework's functionality. The non-transitory computer-readable storage medium has tangibly stored thereon, or tangibly encoded thereon, computer readable instructions that when executed by a device cause at least one processor to perform a method for a DI-based computerized framework for deterministically determining atmospheric data and patterns in/around locations, and leveraging such information for on-demand access and usage on similar sites.

In accordance with one or more embodiments, a system is provided that includes one or more processors and/or computing devices configured to provide functionality in accordance with such embodiments. In accordance with one or more embodiments, functionality is embodied in steps of a method performed by at least one computing device. In accordance with one or more embodiments, program code (or program logic) executed by a processor(s) of a computing device to implement functionality in accordance with one or more such embodiments is embodied in, by and/or on a non-transitory computer-readable medium.

### DESCRIPTIONS OF THE DRAWINGS

The features, and advantages of the disclosure will be apparent from the following description of embodiments as illustrated in the accompanying drawings, in which reference characters refer to the same parts throughout the various views. The drawings are not necessarily to scale, emphasis instead being placed upon illustrating principles of the disclosure:
FIG. 1 is a block diagram of an example configuration within which the systems and methods disclosed herein could be implemented according to some embodiments of the present disclosure;
FIG. 2 is a block diagram illustrating components of an exemplary system according to some embodiments of the present disclosure;
FIG. 3 illustrates an exemplary work flow according to some embodiments of the present disclosure;
FIG. 4 illustrates an exemplary work flow according to some embodiments of the present disclosure;
FIG. 5 illustrates an exemplary work flow according to some embodiments of the present disclosure;
FIG. 6 depicts an exemplary implementation of an architecture according to some embodiments of the present disclosure;
FIG. 7 depicts an exemplary implementation of an architecture according to some embodiments of the present disclosure; and
FIG. 8 is a block diagram illustrating a computing device showing an example of a client or server device used in various embodiments of the present disclosure.

### DETAILED DESCRIPTION

The present disclosure will now be described more fully hereinafter with reference to the accompanying drawings, which form a part hereof, and which show, by way of non-limiting illustration, certain example embodiments. Subject matter may, however, be embodied in a variety of different forms and, therefore, covered or claimed subject matter is intended to be construed as not being limited to any example embodiments set forth herein; example embodiments are provided merely to be illustrative. Likewise, a reasonably broad scope for claimed or covered subject matter is intended. Among other things, for example, subject matter may be embodied as methods, devices, components, or systems. Accordingly, embodiments may, for example, take the form of hardware, software, firmware or any combination thereof (other than software per se). The following detailed description is, therefore, not intended to be taken in a limiting sense.

Throughout the specification and claims, terms may have nuanced meanings suggested or implied in context beyond an explicitly stated meaning. Likewise, the phrase "in one embodiment" as used herein does not necessarily refer to the same embodiment and the phrase "in another embodiment" as used herein does not necessarily refer to a different embodiment. It is intended, for example, that claimed subject matter include combinations of example embodiments in whole or in part.

In general, terminology may be understood at least in part from usage in context. For example, terms, such as "and", "or", or "and/or," as used herein may include a variety of meanings that may depend at least in part upon the context in which such terms are used. Typically, "or" if used to associate a list, such as A, B or C, is intended to mean A, B, and C, here used in the inclusive sense, as well as A, B or C, here used in the exclusive sense. In addition, the term "one or more" as used herein, depending at least in part upon context, may be used to describe any feature, structure, or characteristic in a singular sense or may be used to describe combinations of features, structures or characteristics in a plural sense. Similarly, terms, such as "a," "an," or "the," again, may be understood to convey a singular usage or to convey a plural usage, depending at least in part upon context. In addition, the term "based on" may be understood as not necessarily intended to convey an exclusive set of factors and may, instead, allow for existence of additional factors not necessarily expressly described, again, depending at least in part on context.

The present disclosure is described below with reference to block diagrams and operational illustrations of methods and devices. It is understood that each block of the block diagrams or operational illustrations, and combinations of blocks in the block diagrams or operational illustrations, can be implemented by means of analog or digital hardware and computer program instructions. These computer program instructions can be provided to a processor of a general purpose computer to alter its function as detailed herein, a special purpose computer, ASIC, or other programmable data processing apparatus, such that the instructions, which execute via the processor of the computer or other programmable data processing apparatus, implement the functions/acts specified in the block diagrams or operational block or blocks. In some alternate implementations, the functions/acts noted in the blocks can occur out of the order noted in the operational illustrations. For example, two blocks shown in succession can in fact be executed substantially concurrently or the blocks can sometimes be executed in the reverse order, depending upon the functionality/acts involved.

For the purposes of this disclosure a non-transitory computer readable medium (or computer-readable storage medium/media) stores computer data, which data can include computer program code (or computer-executable instructions) that is executable by a computer, in machine readable form. By way of example, and not limitation, a computer readable medium may include computer readable storage media, for tangible or fixed storage of data, or communication media for transient interpretation of code-containing signals. Computer readable storage media, as used herein, refers to physical or tangible storage (as opposed to signals) and includes without limitation volatile and non-volatile, removable and non-removable media implemented in any method or technology for the tangible storage of information such as computer-readable instructions, data structures, program modules or other data. Computer readable storage media includes, but is not limited to, RAM, ROM, EPROM, EEPROM, flash memory or other solid state memory technology, optical storage, cloud storage, magnetic storage devices, or any other physical or material medium which can be used to tangibly store the desired information or data or instructions and which can be accessed by a computer or processor.

For the purposes of this disclosure the term "server" should be understood to refer to a service point which provides processing, database, and communication facilities. By way of example, and not limitation, the term "server" can refer to a single, physical processor with associated communications and data storage and database facilities, or it can refer to a networked or clustered complex of processors and associated network and storage devices, as well as operating software and one or more database systems and application software that support the services provided by the server. Cloud servers are examples.

For the purposes of this disclosure a "network" should be understood to refer to a network that may couple devices so that communications may be exchanged, such as between a server and a client device or other types of devices, including between wireless devices coupled via a wireless network, for example. A network may also include mass storage, such as network attached storage (NAS), a storage area network (SAN), a content delivery network (CDN) or other forms of computer or machine-readable media, for example. A network may include the Internet, one or more local area networks (LANs), one or more wide area networks (WANs), wire-line type connections, wireless type connections, cellular or any combination thereof. Likewise, sub-networks, which may employ differing architectures or may be compliant or compatible with differing protocols, may interoperate within a larger network.

For purposes of this disclosure, a "wireless network" should be understood to couple client devices with a network. A wireless network may employ stand-alone ad-hoc networks, mesh networks, Wireless LAN (WLAN) networks, cellular networks, or the like. A wireless network may further employ a plurality of network access technologies, including Wi-Fi, Long Term Evolution (LTE), WLAN, Wireless Router mesh, or 2nd, 3rd, 4^{th} or 5^{th} generation (2G, 3G, 4G or 5G) cellular technology, mobile edge computing (MEC), Bluetooth, 802.11b/g/n, or the like. Network access technologies may enable wide area coverage for devices, such as client devices with varying degrees of mobility, for example.

In short, a wireless network may include virtually any type of wireless communication mechanism by which signals may be communicated between devices, such as a client device or a computing device, between or within a network, or the like.

A computing device may be capable of sending or receiving signals, such as via a wired or wireless network, or may be capable of processing or storing signals, such as in memory as physical memory states, and may, therefore, operate as a server. Thus, devices capable of operating as a server may include, as examples, dedicated rack-mounted servers, desktop computers, laptop computers, set top boxes, integrated devices combining various features, such as two or more features of the foregoing devices, or the like.

For purposes of this disclosure, a client (or user, entity, subscriber or customer) device may include a computing device capable of sending or receiving signals, such as via a wired or a wireless network. A client device may, for example, include a desktop computer or a portable device, such as a cellular telephone, a smart phone, a display pager, a radio frequency (RF) device, an infrared (IR) device a Near Field Communication (NFC) device, a Personal Digital Assistant (PDA), a handheld computer, a tablet computer, a phablet, a laptop computer, a set top box, a wearable computer, smart watch, an integrated or distributed device combining various features, such as features of the forgoing devices, or the like.

A client device may vary in terms of capabilities or features. Claimed subject matter is intended to cover a wide range of potential variations, such as a web-enabled client device or previously mentioned devices may include a high-resolution screen (HD or 4K for example), one or more physical or virtual keyboards, mass storage, one or more accelerometers, one or more gyroscopes, global positioning system (GPS) or other location-identifying type capability, or a display with a high degree of functionality, such as a touch-sensitive color 2D or 3D display, for example.

Certain embodiments and principles will be discussed in more detail with reference to the figures. According to some embodiments, the disclosed systems and methods provide advanced computerized mechanisms to monitor, analyze and manage atmospheric conditions with improved accuracy and efficiency. The disclosed framework provides a multifaceted approach that combines sensor networks, data analytics, AI/ML techniques and/or simulation capabilities to create a comprehensive understanding of the vertical air columns above critical assets. As discussed herein, this understanding can be stored as a data structure in a database (e.g., blockchain, for example), which can then be utilized as a basis for training other forms and/or types of sensors. This can improve how sites are managed, and can improve how critical faults, accidents and/or other types of events are predicted, prevented, avoided and/or otherwise stopped from occurring.

According to some embodiments, the disclosed framework can compile, update and manage a secure, robust database that serves as a repository for a vast array of climate and asset data. As discussed herein, the database can employ advanced technologies, such as, for example, blockchain or distributed ledger technologies to ensure data integrity, transparency and immutability. Sensor readings from the vertical air columns, asset information, meteorological data, emission inventories and geographical data, for example, can be integrated and layered within this database, creating a comprehensive and auditable record of atmospheric conditions and their relationship to terrestrial assets.

According to some embodiments, the disclosed framework is configured to develop intelligence from the layered data, as discussed herein. That is, in some embodiments, with the layered data being created and stored in the database, the disclosed framework can operate to extract valuable insights and develop a deep understanding of the vertical air columns above specific assets. As discussed in more detail below, advanced AI/ML (and in some embodiments, large language models (LLMs) models can execute to analyze the complex interplay between atmospheric conditions, asset characteristics and environmental factors, thereby identifying patterns, trends and correlations that may not be immediately apparent to human observers. Such DI-based processing can enable predictive modeling and scenario analysis, allowing devices, systems and/or users to anticipate potential environmental impacts, optimize asset operations and proactively mitigate risks. By continuously learning from the vast amount of data collected, the framework can evolve and refine its intelligence, providing increasingly accurate and actionable insights over time.

According to some embodiments, the disclosed systems and methods can synthesize the determined intelligence to terrestrial sensors and other assets. According to some embodiments, as discussed in more detail below, the disclosed framework can leverage its comprehensive DI-based understanding of atmospheric conditions to optimize sensor placement, calibrate sensor readings, and dynamically adjust sensor configurations to ensure maximum accuracy and efficiency. Moreover, in some embodiments, synthesized intelligence can be integrated into various operational systems, such as emissions control mechanisms, site management platforms, asset maintenance protocols and the like. Thus, by leveraging the holistic understanding of atmospheric conditions and asset performance, the disclosed framework can operate to enable proactive measures to mitigate environmental impacts, improve operational efficiency and ensure regulatory compliance.

According to some embodiments, the disclosed framework can function to generate simulations to replicate vertical column conditions. According to some embodiments, the framework can employ advanced simulation tools to replicate and model the conditions within the vertical air columns by leveraging the layered data, DI and validated models to create virtual representations of atmospheric dynamics, asset operations, and environmental interactions. Such simulations can be for past events, current conditions and/or future/predicted times/dates. As discussed herein, such simulations can enable the exploration of various scenarios, testing hypotheses and evaluations of the potential impacts of different operational strategies or environmental conditions. Such simulations can serve as powerful decision support tools, enabling data-driven decision-making and facilitating the development of proactive mitigation strategies before implementing them in the real world.

Accordingly, as discussed herein, the disclosed systems and methods can provide a synergistic combination of a layered data repository, DI-based computational analysis, sensor integration and simulation capabilities, which can provide an interactive holistic system for monitoring, analyzing and managing atmospheric conditions at specific locations. Such framework can provide unprecedented insights, enabling systems, sites, assets and/or users to make informed decisions, optimize asset operations and proactively mitigate environmental impacts.

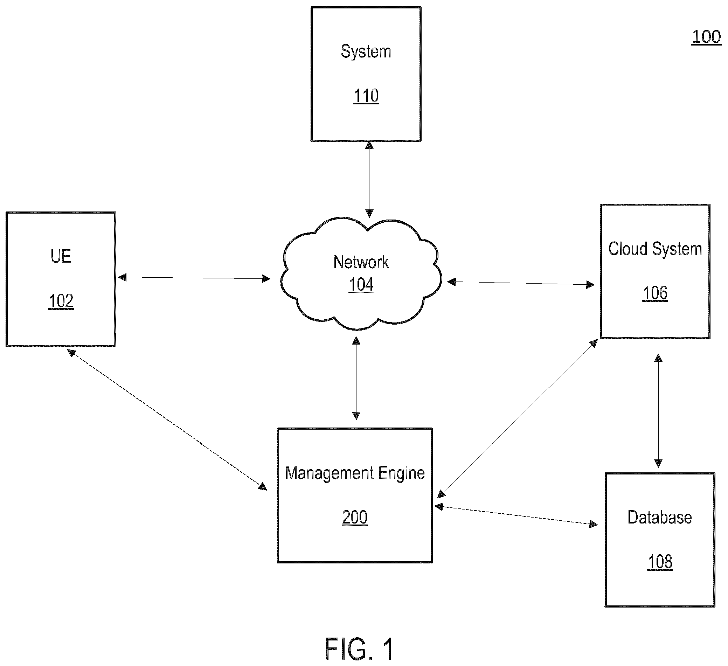

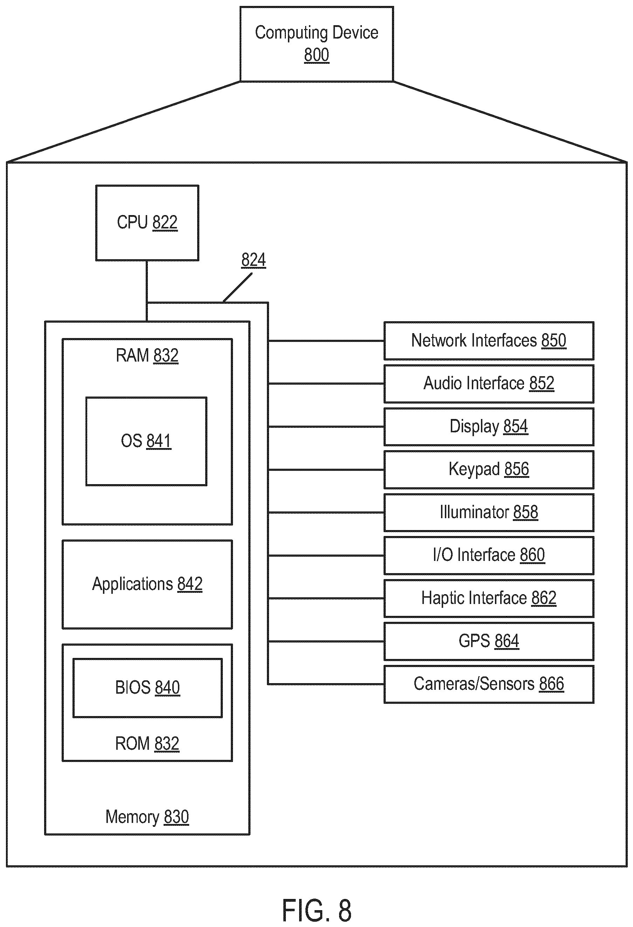

With reference to FIG. 1, system 100 is depicted which includes user equipment (UE) 102 (e.g., a client device, as mentioned above and discussed below in relation to FIG. 8), system 110, network 104, cloud system 106, database 108 and management engine 200.

It should be understood that while system 100 is depicted as including such components, it should not be construed as limiting, as one of ordinary skill in the art would readily understand that varying numbers of UEs, devices, users/entities, systems, cloud systems, databases and networks can be utilized; however, for purposes of explanation, system 100 is discussed in relation to the example depiction in FIG. 1.

According to some embodiments, UE 102 can be any type of device, such as, but not limited to, a mobile phone, tablet, laptop, Internet of Things (IoT) device, autonomous machine, sensor, and any other device equipped with functionality for connecting to a network and performing computational activities for collecting information and/or interacting with other devices.

In some embodiments, UE 102 can be associated with a location, site, tool, sensor, asset and the like, and can correspond to, but not be limited to, but not limited to, oil and gas facilities, industrial plants and factories, mining operations, agricultural operations, waste management, transportation, power generation facilities, and the like, or some combination thereof.

In some embodiments, for example, UE 102 can be a sensor for, but not limited to, well pad, drilling rig, production site, processing site, pipeline, storage and/or extraction facility, and the like, at an oil and gas facility. Such sensors, for example, can be, but are not limited to, methane sensors, volatile organic compound (VOC) sensors, particulate matter (PM) sensors, combustion gas sensors, meteorological sensors, optical gas imaging (OGI) sensors, ambient air quality sensors, acoustic sensors, and the like. Thus, while it should not be construed as limiting, such sensors are examples of types of sensors UE 102 can be and/or be associated with, such that any type of known or to be known type of UE/sensor can be utilized for the disclosed systems and methods without departing from the scope of the instant disclosure.

According to some embodiments, system 110 can correspond to any type of device (or UE, as discussed above), computer system, electronic platform, web portal, electronically hosted network resource, and the like, or some combination thereof. In some embodiments, for example, system 100 can correspond to a third party company (e.g., oil and gas company, for example) for which a user of UE 102 is electronically interacting with to conduct business and/or manage their jobsite.

In some embodiments, network 104 can be any type of network, such as, but not limited to, a wireless network, cellular network, the Internet, and the like (as discussed above). Network 104 facilitates connectivity of the components of system 100, as illustrated in FIG. 1.

According to some embodiments, cloud system 106 may be any type of cloud operating platform and/or network based system upon which applications, operations, and/or other forms of network resources may be located. For example, system 106 may be a service provider and/or network provider from where services and/or applications may be accessed, sourced or executed from. For example, system 106 can represent the cloud-based architecture associated with a proprietary system provider, which has associated network resources hosted on the internet or private network (e.g., network 104), which enables (via engine 200) the climate and/or asset management and monitoring discussed herein.

In some embodiments, cloud system 106 may include a server(s) and/or a database of information which is accessible over network 104. In some embodiments, a database 108 of cloud system 106 may store a dataset of data and metadata associated with local and/or network information related to a user(s) of UE 102/system 110 and the UE 102/system 110, and the services and applications provided by cloud system 106 and/or management engine 200.

In some embodiments, for example, cloud system 106 can provide a private/proprietary management platform, whereby engine 200, discussed *infra,* corresponds to the novel functionality system 106 enables, hosts and provides to a network 104 and other devices/platforms operating thereon.

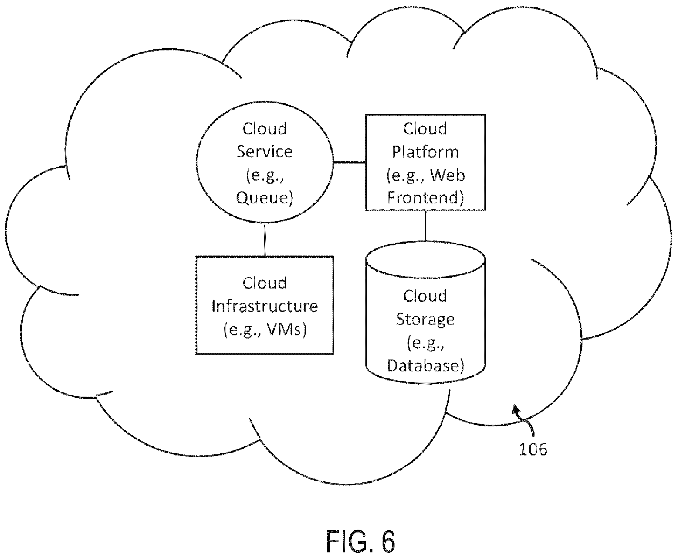

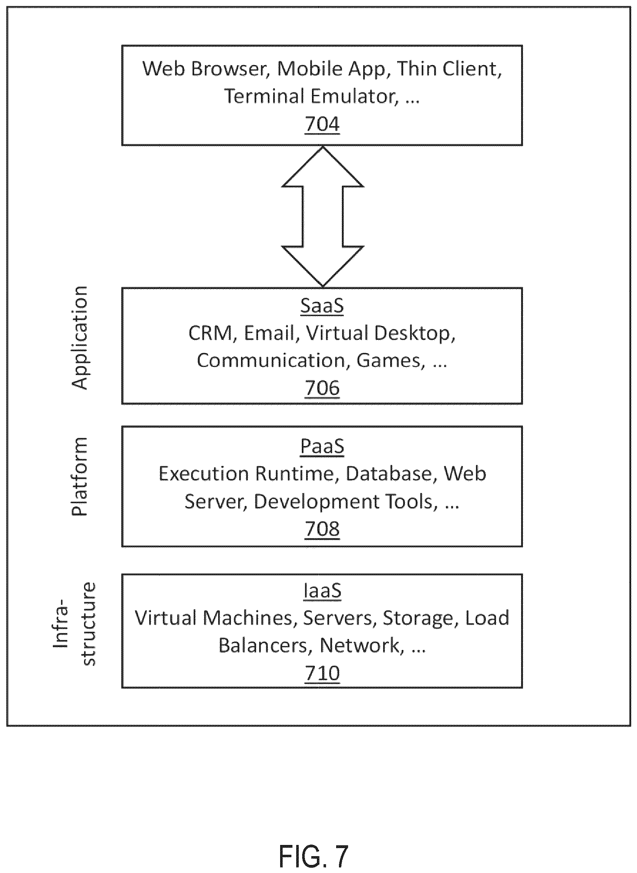

Turning to FIG. 6 and FIG. 7, in some embodiments, the exemplary computer-based systems/platforms, the exemplary computer-based devices, and/or the exemplary computer-based components of the present disclosure may be specifically configured to operate in a cloud computing/architecture 106 such as, but not limiting to: infrastructure a service (IaaS) 710, platform as a service (PaaS) 708, and/or software as a service (SaaS) 706 using a web browser, mobile app, thin client, terminal emulator or other endpoint 704. FIG. 6 and FIG. 7 illustrate schematics of non-limiting implementations of the cloud computing/architecture(s) in which the exemplary computer-based systems for administrative customizations and control of network-hosted application program interfaces (APIs) of the present disclosure may be specifically configured to operate.

Turning back to FIG. 1, according to some embodiments, database 108 may correspond to a data storage for a platform (e.g., a network hosted platform, such as cloud system 106, as discussed *supra*) or a plurality of platforms. Database 108 may receive storage instructions/requests from, for example, engine 200 (and associated microservices), which may be in any type of known or to be known format, such as, for example, standard query language (SQL). According to some embodiments, database 108 may correspond to any type of known or to be known storage, for example, a memory or memory stack of a device, a distributed ledger of a distributed network (e.g., blockchain, for example), a look-up table (LUT), and/or any other type of secure data repository

Management engine 200, as discussed above and further below in more detail, can include components for the disclosed functionality. According to some embodiments, management engine 200 may be a special purpose machine or processor, and can be hosted by a device on network 104, within cloud system 106 and/or on UE 102. In some embodiments, engine 200 may be hosted by a server and/or set of servers associated with cloud system 106.

According to some embodiments, as discussed in more detail below, management engine 200 may be configured to implement and/or control a plurality of services and/or microservices, where each of the plurality of services/microservices are configured to execute a plurality of workflows associated with performing the disclosed functionality. Non-limiting embodiments of such workflows are provided below.

According to some embodiments, as discussed above, management engine 200 may function as an application provided by cloud system 106. In some embodiments, engine 200 may function as an application installed on a server(s), network location and/or other type of network resource associated with cloud system 106. In some embodiments, engine 200 may function as an application installed and/or executing on UE 102. In some embodiments, such application may be a web-based application accessed by UE 102 and/or devices over network 104 from cloud system 106. In some embodiments, engine 200 may be configured and/or installed as an augmenting script, program or application (e.g., a plug-in or extension) to another application or program provided by cloud system 106 and/or executing on UE 102.

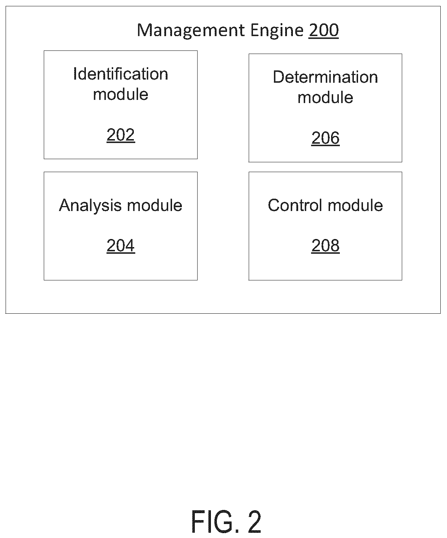

As illustrated in FIG. 2, according to some embodiments, management engine 200 includes identification module 202, analysis module 204; determination module 206 and control module 208. It should be understood that the engine(s) and modules discussed herein are non-exhaustive, as additional or fewer engines and/or modules (or sub-modules) may be applicable to the embodiments of the systems and methods discussed. More detail of the operations, configurations and functionalities of engine 200 and each of its modules, and their role within embodiments of the present disclosure will be discussed below.

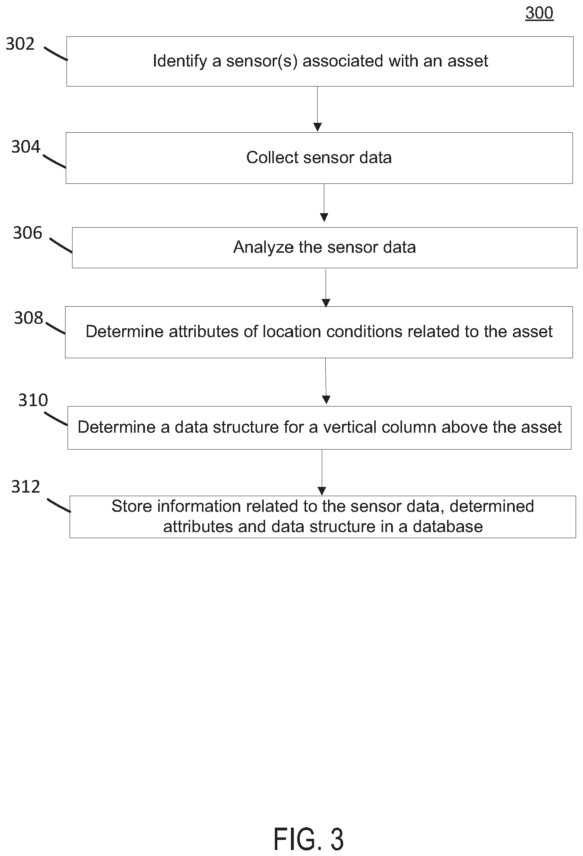

Turning to FIG. 3, Process 300 provides non-limiting example embodiments for the disclosed systems and methods. Process 300 provides non-limiting example embodiments for the compilation of the layered data for an asset at a location within a secure data store (e.g., blockchain, for example), as discussed herein.

According to some embodiments, Steps 302 and 304 of Process 300 can be performed by identification module 202 of management engine 200; Step 306 can be performed by analysis module 204; Steps 308 and 310 can be performed by determination module 206; and Step 312 can be performed by control module 208.

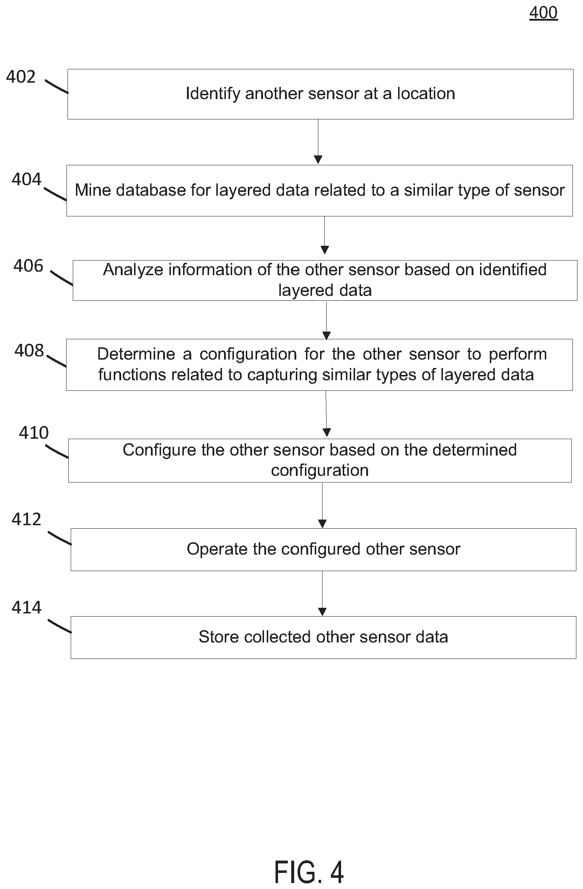

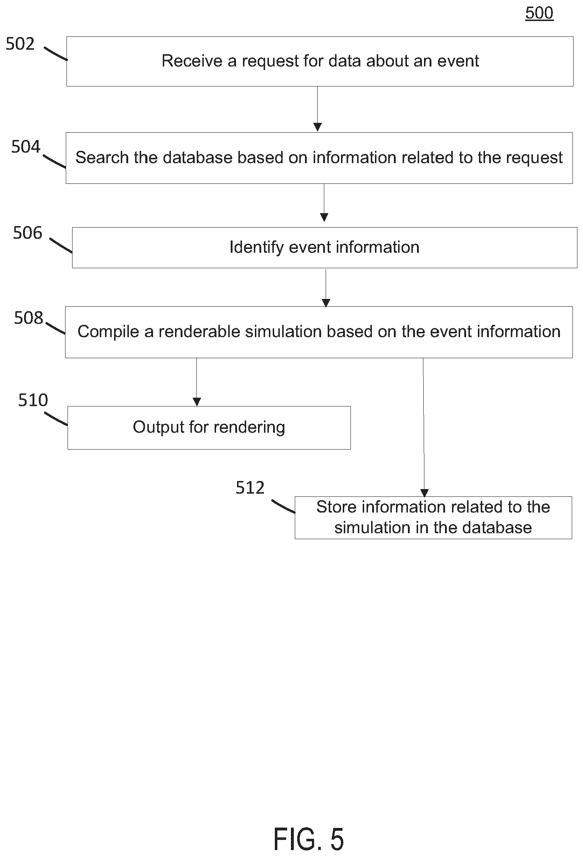

According to some embodiments, Process 300 begins with Step 302 where a sensor(s) associated with an asset is identified. For the purposes simplicity, a single sensor and single asset at a location will be discussed with reference to FIG. 3 (and FIGs. 4 and 5, *infra*); however, it should not be construed as limiting, as one of ordinary skill in the art would readily recognize that the steps performed in Process 300 (and Processes 400 and 500, *infra*) can be performed for multiple sensors for an asset, for multiple assets and/or multiple locations. For example, Step 302 can involve identifying a methane sensor for an oil rig at a drilling site.

In Step 304, engine 200 can operate to collect sensor data. Such collection can correspond to a criteria, which can be based on, but not limited to, a time (or time period), date, request, detection of an event (e.g.,, triggered fault, for example), and the like, or some combination thereof. In some embodiments, the collection can occur continuously and/or periodically (e.g., per the criteria being satisfied).

According to some embodiments, as discussed above, such sensor data can include data related to, but not limited to, a location, identifier (ID), time, date, type of data (that pertains to the type of sensor - for example, a methane sensor can collect methane measurements), and the like. In some embodiments, such collected sensor data can be stored in database 108, as discussed *supra.*

In Step 306, engine 200 can analyze the collected sensor data. According to some embodiments, engine 200 can implement any type of known or to be known computational analysis technique, algorithm, mechanism or technology to analyze the collected sensor data from Step 304.

In some embodiments, engine 200 may include a specific trained AI/ML model, a particular machine learning model architecture, a particular machine learning model type (e.g., convolutional neural network (CNN), recurrent neural network (RNN), autoencoder, support vector machine (SVM), and the like), or any other suitable definition of a machine learning model or any suitable combination thereof.

In some embodiments, engine 200 may be configured to utilize one or more AI/ML techniques chosen from, but not limited to, computer vision, feature vector analysis, decision trees, boosting, support-vector machines, neural networks, nearest neighbor algorithms, Naive Bayes, bagging, random forests, logistic regression, and the like.

In some embodiments and, optionally, in combination of any embodiment described above or below, a neural network technique may be one of, without limitation, feedforward neural network, radial basis function network, recurrent neural network, convolutional network (e.g., U-net) or other suitable network. In some embodiments and, optionally, in combination of any embodiment described above or below, an implementation of Neural Network may be executed as follows:
a. define Neural Network architecture/model,
b. transfer the input data to the neural network model,
c. train the model incrementally,
d. determine the accuracy for a specific number of timesteps,
e. apply the trained model to process the newly-received input data,
f. optionally and in parallel, continue to train the trained model with a predetermined periodicity.

In some embodiments and, optionally, in combination of any embodiment described above or below, the trained neural network model may specify a neural network by at least a neural network topology, a series of activation functions, and connection weights. For example, the topology of a neural network may include a configuration of nodes of the neural network and connections between such nodes. In some embodiments and, optionally, in combination of any embodiment described above or below, the trained neural network model may also be specified to include other parameters, including but not limited to, bias values/functions and/or aggregation functions. For example, an activation function of a node may be a step function, sine function, continuous or piecewise linear function, sigmoid function, hyperbolic tangent function, or other type of mathematical function that represents a threshold at which the node is activated. In some embodiments and, optionally, in combination of any embodiment described above or below, the aggregation function may be a mathematical function that combines (e.g., sum, product, and the like) input signals to the node. In some embodiments and, optionally, in combination of any embodiment described above or below, an output of the aggregation function may be used as input to the activation function. In some embodiments and, optionally, in combination of any embodiment described above or below, the bias may be a constant value or function that may be used by the aggregation function and/or the activation function to make the node more or less likely to be activated.

In Step 308, based on the analysis from Step 306, engine 200 can determine attributes (or features or characteristics) of location conditions related to the asset. Such location conditions can include geospatial information, atmospheric information, climate information, meteorological information, and the like.

According to some embodiments, such attributes can correspond to, but not be limited to, emission rates and concentrations, leak detection and localization, air quality assessments, meteorological conditions, emissions source attribution, dispersion modeling, operational insights, environmental impact assessments, compliance and baseline and/or trend analysis, and the like, or some combination thereof.

Accordingly, by way of a non-limiting example, in some embodiments, the deployment of atmospheric sensors at jobsites and/or near assets enables the determination of a wide range of critical attributes through the analysis of collected data. Such attributes can include, but not be limited to, emission rates and concentrations of methane, VOCs, PM, combustion gases, and other air pollutants, facilitating the identification and quantification of emission sources. Leak detection and localization capabilities can aid in pinpointing leak sources, estimating leak rates, and assessing overall air quality in compliance with regulatory standards. Meteorological conditions, such as, for example, wind speed, direction, temperature, humidity and atmospheric pressure can be monitored, enabling dispersion modeling and impact predictions on nearby communities or ecosystems. Source attribution analyses differentiate between on-site and off-site emission sources, while operational insights help identify inefficiencies, monitor emission control system effectiveness, and evaluate mitigation measure impacts.

In some embodiments, such determined attributes can be stored in database 108, as discussed *supra.*

In Step 310, engine 200 can generate a data structure for a vertical column above the asset. The data structure, which can be an electronic data object or item, and/or an executable file or set of executable instructions that includes the information for the vertical column above the asset, which is based on the determined attributes and sensor data that was analyzed, as discussed above.

According to some embodiments, as discussed herein, a vertical column above an asset can refer to the vertical profile and/or the atmospheric conditions extending upwards from the surface level of the asset and/or facility (or location) being monitored (e.g., to a predetermined value - e.g., 10,000 feet, for example; and being *n* value wide (e.g., a value of a column's diameter that enables the capture of atmospheric layer values that can impact the asset), for example). Such vertical column can encompass the various layers of the atmosphere, from the ground level to higher altitudes, and provides a comprehensive understanding of the atmospheric dynamics and potential impacts associated with the asset's operations or emissions.

In some embodiments, the vertical column data structure can include information related to, but not limited to, atmospheric layers (e.g., different atmospheric layers, such as the troposphere, stratosphere, and potentially the lower mesosphere, depending on the altitude range being monitored); emission dispersion (e.g., the vertical column captures the dispersion patterns of emissions and/or pollutants released from the asset, including their vertical transport, mixing and potential interactions with atmospheric conditions at various altitudes); meteorological profiles (e.g., within the vertical column, meteorological parameters such as temperature, pressure, humidity, wind speed and wind direction can vary significantly with altitude, influencing the behavior and transport of emissions); chemical transformations (e.g., the vertical column can provide insights into the chemical transformations and reactions that emissions or pollutants may undergo as they interact with atmospheric constituents at different altitudes, potentially leading to the formation of secondary pollutants); inversion layers (e.g., atmospheric inversion layers, where temperature increases with altitude, can trap emissions or pollutants within the vertical column, leading to higher concentrations near the surface); vertical mixing and stratification- (e.g., the degree of vertical mixing or stratification within the column can affect the distribution and concentration of emissions or pollutants at different altitudes); long-range transport (e.g., monitoring the vertical column can help understand the potential for long-range transport of emissions or pollutants, as atmospheric conditions at higher altitudes can influence their movement over longer distances); and the like, or some combination thereof.

And, in Step 312, engine 200 can store the information as layered data that is related to the sensor data, determined attributes and/or data structure in the database (e.g., database 108). For example, the generated vertical column data structure can be stored in database 108, which as discussed below, can be used to train another sensor on how to collect similar data.

In some embodiments, the information can be stored in a layered manner; in other words, the stored information can be stored in a structural manner via multiple interconnected layers/levels, where each layer/level can correspond to a type of data (e.g., sensor data, vertical column and/or attributes, for example) and/or granularity.

In some embodiments, as mentioned above, database 108 may correspond to a distributed ledger of a distributed network. In some embodiments, the distributed network may include a plurality of distributed network nodes, where each distributed network node includes and/or corresponds to a computing device associated with at least one entity (e.g., the entity associated with cloud system 106, for example, discussed *supra*)*.* In some embodiments, each distributed network node may include at least one distributed network data store configured to store distributed network-based data objects for the at least one entity. For example, database 108 may correspond to a blockchain, where the distributed network-based data objects can include, but are not limited to, account information, medical information, entity identifying information, wallet information, device information, network information, credentials, security information, permissions, identifiers, smart contracts, transaction history, and the like, or any other type of known or to be known data/metadata related to an entity's and/or user's information, structure, business and/or legal demographics, *inter alia.*

In some embodiments, a blockchain may include one or more private and/or private-permissioned cryptographically-protected, distributed databases, such as, without limitation, a blockchain (distributed ledger technology), Ethereum (Ethereum Foundation, Zug, Switzerland), and/or other similar distributed data management technologies. For example, as utilized herein, the distributed database(s), such as distributed ledgers, ensure the integrity of data by generating a digital chain of data blocks linked together by cryptographic hashes of the data records in the data blocks. For example, a cryptographic hash of at least a portion of data records within a first block, and, in some cases, combined with a portion of data records in previous blocks is used to generate the block address for a new digital identity block succeeding the first block. As an update to the data records stored in the one or more data blocks, a new data block is generated containing respective updated data records and linked to a preceding block with an address based upon a cryptographic hash of at least a portion of the data records in the preceding block. In other words, the linked blocks form a blockchain that inherently includes a traceable sequence of addresses that may be used to track the updates to the data records contained therein. The linked blocks (or blockchain) may be distributed among multiple network nodes within a computer network such that each node may maintain a copy of the blockchain. Malicious network nodes attempting to compromise the integrity of the database must recreate and redistribute the blockchain faster than the honest network nodes, which, in most cases, is computationally infeasible. In other words, data integrity is guaranteed by the virtue of multiple network nodes in a network having a copy of the same blockchain. In some embodiments, as utilized herein, a central trust authority for sensor data management may not be needed to vouch for the integrity of the distributed database hosted by multiple nodes in the network.

In some embodiments, the exemplary distributed blockchain-type ledger implementations of the present disclosure with associated devices are configured to utilize smart contracts that are computer processes that facilitate, verify and/or enforce negotiation and/or performance of one or more particular activities among users/parties. For example, an exemplary smart contract may be configured to be partially or fully self-executing and/or self-enforcing. In some embodiments, the exemplary inventive asset-tokenized distributed blockchain-type ledger implementations of the present disclosure may utilize smart contract architecture that may be implemented by replicated asset registries and contract execution using cryptographic hash chains and Byzantine fault tolerant replication. For example, each node in a peer-to-peer network or blockchain distributed network may act as a title registry and escrow, thereby executing changes of ownership and implementing sets of predetermined rules that govern transactions on the network. For example, each node may also check the work of other nodes and in some cases, as noted above, function as miners or validators.

Turning to FIG. 4, Process 400 provides non-limiting example embodiments for the synthesis of other sensors/sites based on the stored information from Process 300, discuss *supra.*

According to some embodiments, Steps 402 and 404 of Process 400 can be performed by identification module 202 of management engine 200; Step 406 can be performed by analysis module 204; Step 408 can be performed by determination module 206; and Steps 410-414 can be performed by control module 208.

In some embodiments, Process 400 can begin with Step 402 where another sensor at a location is identified. Step 402 can function in a similar manner as Step 302 of Process 300, discussed *supra.*

In some embodiments, Step 402 can involve identification of sensor information, which can include, but is not limited to, the ID of the sensor, location (e.g., where at a jobsite/facility), which jobsite/facility, position on an asset, a type and/or ID of the associated asset, and the like.

In Step 404, engine 200 can compile a search request to mine database 108 based on the information related to the other sensor. In some embodiments, Step 404 can involve a search request/query that is utilized to parse the records stored in the database 108 and identify stored information (as discussed above in Process 300, *supra*) that relates to the sensor information of the other sensor (identified in Step 402). In some embodiments, such search can involve AI/ML analysis, in that a similarity analysis can be performed to identify stored data that satisfies a similarity threshold.

Thus, in some embodiments, Step 404 can involve mining the database 108 for stored data about a vertical column above a sensor that is the same type and in a similar location as the other sensor identified in Step 404. For example, the other sensor can be a terrestrial methane sensor, while the sensor data identified from the mining of the database can be an airborne methane sensor.

In Step 406, engine 200 can perform computational analysis operations of the sensor information for the other sensor (from Step 402) based on the retrieved data from the mining (from Step 404). In some embodiments, such analysis can be performed via the AI/ML techniques discussed above in Process 300.

In Step 408, engine 200 can determine, based on the analysis from Step 406, a configuration for the other sensor to perform functions related to capturing similar types of layered data. That is, a configuration that enables the sensor to operate to collect data that is capable of generating vertical column information for the asset associated with the other sensor, for example. In some embodiments, the configuration can be compiled as a data structure, which can be an executable file or set of instructions that can cause modifications to how the other sensor can operate, as discussed in more detail below.

In Step 410, engine 200 can execute the determined configuration (from Step 408) to configure the other sensor. Such configuration can cause the sensor to perform operations (e.g., collect types and/or quantities of data, respond to requests and/or operate in a novel manner) that are non-native to the sensor prior to the configuration.

By way of a non-limiting example, with reference to the other sensor being a terrestrial sensor and the collected data from the mining being related to an airborne sensor, the synthesis of intelligence from airborne sensors to terrestrial sensors can involve a process of data integration, analysis, and optimization to enhance the performance and capabilities of ground-based monitoring systems.

According to some embodiments, the mined data can include, for example, measurements of greenhouse gases, air pollutants, aerosols, meteorological parameters, and other atmospheric properties (e.g., where the airborne sensor can be, for example, mounted on satellites, aircraft, drones, or weather balloons, to collect atmospheric data at various altitudes).

In some embodiments, the data collected from airborne sensors can be processed and analyzed using advanced techniques, such as AI/ML algorithms, atmospheric models, and data fusion methods. This analysis can reveal patterns, trends, and insights into atmospheric processes, emission sources, and dispersion patterns. Examples of such processing are discussed above in relation to Process 300, discussed *supra.*

In some embodiments, engine 200 can then perform an integration with terrestrial sensor data, where the intelligence derived from airborne sensor data can be integrated with data from terrestrial sensors, such as, for example, ground-based air quality monitoring stations, methane detectors, or weather stations. Such integration combines the broad, regional perspective provided by airborne sensors with the localized, high-resolution data from terrestrial sensors.

In some embodiments, the integrated data from airborne and terrestrial sensors can be used to calibrate and validate atmospheric models and predictive algorithms. Such models can be used to simulate and predict atmospheric conditions, emissions, and their impacts on a localized scale, accounting for factors such as terrain, urbanization, and local emission sources, as discussed *infra.*

In some embodiments, the intelligence gained from the integrated data and models can be used to optimize the placement and configuration of terrestrial sensors. This can involve identifying optimal locations for sensor deployment, adjusting sensor parameters (e.g., sensitivity, sampling frequency), or reconfiguring sensor networks to improve coverage and accuracy.

According to some embodiments, engine 200 can perform and/or enable real-time monitoring and decision support, where the synthesized intelligence from airborne and terrestrial sensors can be used for real-time monitoring and decision support systems. Such systems can provide alerts and notifications for potential emission events, air quality concerns, or regulatory compliance issues, enabling timely and informed decision-making.

In some embodiments, the performance of the terrestrial sensors and monitoring systems can be continuously evaluated and improved based on the feedback from airborne sensor data and atmospheric models. Such feedback loop allows for the refinement of algorithms, sensor configurations, and monitoring strategies, leading to a more comprehensive and accurate understanding of atmospheric conditions.

As such, as discussed herein, by synthesizing intelligence from a known sensor to another unknown sensor (e.g., airborne and terrestrial sensors, for example), an improved multi-scale understanding of atmospheric processes can be attained, enabling more effective environmental monitoring, emission mitigation strategies, and regulatory compliance efforts.

Thus, in Step 412, engine 200 can operate the configured other sensor, which can operate in a similar manner as discussed above with respect to the steps of Process 300 (e.g., the operation of Step 412 can involve the performance of the steps of Process 300 for the configured other sensor). And, in Step 414, the data generated from operation of the configured other sensor can be stored, in a similar manner as discussed above, in database 108.

Turning to FIG. 5, Process 500 provides non-limiting example embodiments for the generation of a simulation, as discussed above.

According to some embodiments, Steps 502-506 of Process 500 can be performed by identification module 202 of management engine 200; and Steps 508-512 can be performed by control module 208.

In some embodiments, Process 500 can begin with Step 502 where a request for data about an event can be received. In some embodiments, the request can be automatically generated as part of an audit process and/or associated with a predetermined time period to perform a system check. In some embodiments, the request can be provided by an operator or third party (e.g. government body).

In some embodiments, the request can be related to the occurrence of an event, occurrence of a non-event (no activities of a particular type were detected and wanting to double check the status of the sensors as such time - for example - no leaks by the asset), and the like. In some embodiments, the request can include information related to, but not limited to, an ID of a sensor, ID of an asset, ID of a location, type of measurement, quantity/value of measurement, time, date, atmospheric layer, and the like, or some combination thereof.

In Step 504, engine 200 can perform a search of the database (e.g., database 108) based on the request, where the information in the request can be used to compile a search query used to parse and mine the database to extract the relevant data. Such search can be performed in a similar manner as discussed above in relation to Process 400 (e.g., Step 404, *inter alia*)*.*

In Step 506, based on the search in Step 504, engine 200 can identify event information that was requested. For example, vertical column information above a set of assets at a location can be provided as a result in response to a request for such information related to time X and location Y.

In Step 508, engine 200 can compile a renderable simulation based on the identified information. In some embodiments, as discussed above, the vertical column information can be stored as an executable data structure, whereby upon execution, a visual simulation or rendering of captured data (e.g., image, for example) frames can be generated that illustrates the measured values from the sensors for that asset/location. In other words, the simulation can replicate how the layered data (included within the event information) is realized within the vertical column above an asset(s). Thus, in Step 508, an executable file can be prepared, compiled and/or primed for output, as in Step 510, which can be provided via a message to the requesting device/user and/or displayed within a user interface (UI) of such device.

And, in Step 512, engine 200 can store the information for the simulation in the database, which can supplement the retrieved data, which can enhance how the data can be analyzed and/or increase efficiency in which the data can be retrieved from subsequent requests, where such storage can include the simulation compiled in Step 508, as well as the information from the request (from Step 502) and/or the event information (from Step 506).

According to some embodiments, the disclosed framework can employ quantum technologies for the performance of atmospheric monitoring by providing enhanced capabilities for sensing, data processing and modeling, as discussed herein

In some embodiments, the disclosed framework can perform quantum sensing, where quantum sensors can be used for highly sensitive and accurate measurements of atmospheric parameters such as greenhouse gas concentrations, air pollutants, wind speed, and temperature. Quantum-based spectroscopy techniques, like quantum cascade lasers, can be employed for high-precision detection and quantification of atmospheric gases and trace molecules. Quantum magnetometers and gravimeters can be used for mapping Earth's magnetic and gravitational fields, which can provide insights into atmospheric dynamics and composition.

In some embodiments, the disclosed framework can perform quantum-enhanced remote sensing, where quantum entanglement and quantum correlations can be exploited to improve the resolution and sensitivity of remote sensing instruments used for atmospheric monitoring from satellites or aircraft. Quantum radar and quantum lidar techniques can enhance the detection and characterization of atmospheric particles, clouds, and aerosols.

In some embodiments, the disclosed framework can perform quantum computing and simulation, where quantum computers can be used to simulate and model complex atmospheric processes and interactions with unprecedented accuracy and speed, enabling more detailed and reliable atmospheric predictions. Quantum algorithms and quantum ML techniques can be applied to process and analyze vast amounts of atmospheric data, extracting patterns and insights that are difficult or impossible to obtain with classical computing methods.

In some embodiments, quantum communication and networking, where quantum communication protocols, such as quantum key distribution, can be used to securely transmit and share atmospheric data between monitoring stations, satellites, and ground-based facilities, ensuring data integrity and security. Quantum networks can enable the efficient and secure distribution of atmospheric data and models across different locations and organizations involved in atmospheric monitoring.

In some embodiments, the disclosed framework can perform quantum-inspired optimization and decision-making, where quantum-inspired optimization algorithms can be employed to optimize the placement and configuration of atmospheric monitoring sensors, maximizing coverage and minimizing resource requirements. Quantum decision-making frameworks can assist in making informed decisions based on atmospheric data, balancing multiple objectives such as environmental impact, economic considerations, and regulatory compliance.

Accordingly, the above discussed systems and methods can employ known or to be known quantum functionalities and capabilities to improve how the system operates without departing from the scope of the instant disclosure.

FIG. 8 is a schematic diagram illustrating a client device showing an example embodiment of a client device that may be used within the present disclosure. Client device 800 may include many more or less components than those shown in FIG. 8. However, the components shown are sufficient to disclose an illustrative embodiment for implementing the present disclosure. Client device 800 may represent, for example, UE 102 discussed above at least in relation to FIG. 1.

As shown in the figure, in some embodiments, Client device 800 includes a processing unit (CPU) 822 in communication with a mass memory 830 via a bus 824. Client device 800 also includes a power supply 826, one or more network interfaces 850, an audio interface 852, a display 854, a keypad 856, an illuminator 858, an input/output interface 860, a haptic interface 862, an optional global positioning systems (GPS) receiver 864 and a camera(s) or other optical, thermal or electromagnetic sensors 866. Device 800 can include one camera/sensor 866, or a plurality of cameras/sensors 866, as understood by those of skill in the art. Power supply 826 provides power to Client device 800.

Client device 800 may optionally communicate with a base station (not shown), or directly with another computing device. In some embodiments, network interface 850 is sometimes known as a transceiver, transceiving device, or network interface card (NIC).

Audio interface 852 is arranged to produce and receive audio signals such as the sound of a human voice in some embodiments. Display 854 may be a liquid crystal display (LCD), gas plasma, light emitting diode (LED), or any other type of display used with a computing device. Display 854 may also include a touch sensitive screen arranged to receive input from an object such as a stylus or a digit from a human hand.

Keypad 856 may include any input device arranged to receive input from a user. Illuminator 858 may provide a status indication and/or provide light.

Client device 800 also includes input/output interface 860 for communicating with external. Input/output interface 860 can utilize one or more communication technologies, such as USB, infrared, Bluetooth^{™}, or the like in some embodiments. Haptic interface 862 is arranged to provide tactile feedback to a user of the client device.

Optional GPS transceiver 864 can determine the physical coordinates of Client device 800 on the surface of the Earth, which typically outputs a location as latitude and longitude values. GPS transceiver 864 can also employ other geo-positioning mechanisms, including, but not limited to, triangulation, assisted GPS (AGPS), E-OTD, CI, SAI, ETA, BSS or the like, to further determine the physical location of client device 800 on the surface of the Earth. In one embodiment, however, Client device 800 may through other components, provide other information that may be employed to determine a physical location of the device, including for example, a MAC address, Internet Protocol (IP) address, or the like.

Mass memory 830 includes a RAM 832, a ROM 834, and other storage means. Mass memory 830 illustrates another example of computer storage media for storage of information such as computer readable instructions, data structures, program modules or other data. Mass memory 830 stores a basic input/output system ("BIOS") 840 for controlling low-level operation of Client device 800. The mass memory also stores an operating system 841 for controlling the operation of Client device 800.

Memory 830 further includes one or more data stores, which can be utilized by Client device 800 to store, among other things, applications 842 and/or other information or data. For example, data stores may be employed to store information that describes various capabilities of Client device 800. The information may then be provided to another device based on any of a variety of events, including being sent as part of a header (e.g., index file of the HLS stream) during a communication, sent upon request, or the like. At least a portion of the capability information may also be stored on a disk drive or other storage medium (not shown) within Client device 800.

Applications 842 may include computer executable instructions which, when executed by Client device 800, transmit, receive, and/or otherwise process audio, video, images, and enable telecommunication with a server and/or another user of another client device. Applications 842 may further include a client that is configured to send, to receive, and/or to otherwise process gaming, goods/services and/or other forms of data, messages and content hosted and provided by the platform associated with engine 200 and its affiliates.

As used herein, the terms "computer engine" and "engine" identify at least one software component and/or a combination of at least one software component and at least one hardware component which are designed/programmed/configured to manage/control other software and/or hardware components (such as the libraries, software development kits (SDKs), objects, and the like).

Examples of hardware elements may include processors, microprocessors, circuits, circuit elements (e.g., transistors, resistors, capacitors, inductors, and so forth), integrated circuits, application specific integrated circuits (ASIC), programmable logic devices (PLD), digital signal processors (DSP), field programmable gate array (FPGA), logic gates, registers, semiconductor device, chips, microchips, chip sets, and so forth. In some embodiments, the one or more processors may be implemented as a Complex Instruction Set Computer (CISC) or Reduced Instruction Set Computer (RISC) processors; x86 instruction set compatible processors, multi-core, or any other microprocessor or central processing unit (CPU). In various implementations, the one or more processors may be dual-core processor(s), dual-core mobile processor(s), and so forth.

Computer-related systems, computer systems, and systems, as used herein, include any combination of hardware and software. Examples of software may include software components, programs, applications, operating system software, middleware, firmware, software modules, routines, subroutines, functions, methods, procedures, software interfaces, application program interfaces (API), instruction sets, computer code, computer code segments, words, values, symbols, or any combination thereof. Determining whether an embodiment is implemented using hardware elements and/or software elements may vary in accordance with any number of factors, such as desired computational rate, power levels, heat tolerances, processing cycle budget, input data rates, output data rates, memory resources, data bus speeds and other design or performance constraints.

For the purposes of this disclosure a module is a software, hardware, or firmware (or combinations thereof) system, process or functionality, or component thereof, that performs or facilitates the processes, features, and/or functions described herein (with or without human interaction or augmentation). A module can include sub-modules. Software components of a module may be stored on a computer readable medium for execution by a processor. Modules may be integral to one or more servers, or be loaded and executed by one or more servers. One or more modules may be grouped into an engine or an application.

One or more aspects of at least one embodiment may be implemented by representative instructions stored on a machine-readable medium which represents various logic within the processor, which when read by a machine causes the machine to fabricate logic to perform the techniques described herein. Such representations, known as "IP cores," may be stored on a tangible, machine readable medium and supplied to various customers or manufacturing facilities to load into the fabrication machines that make the logic or processor. Of note, various embodiments described herein may, of course, be implemented using any appropriate hardware and/or computing software languages (e.g., C++, Objective-C, Swift, Java, JavaScript, Python, Perl, QT, and the like).

For example, exemplary software specifically programmed in accordance with one or more principles of the present disclosure may be downloadable from a network, for example, a website, as a stand-alone product or as an add-in package for installation in an existing software application. For example, exemplary software specifically programmed in accordance with one or more principles of the present disclosure may also be available as a client-server software application, or as a web-enabled software application. For example, exemplary software specifically programmed in accordance with one or more principles of the present disclosure may also be embodied as a software package installed on a hardware device.

For the purposes of this disclosure the term "user", "subscriber" "consumer" or "customer" should be understood to refer to a user of an application or applications as described herein and/or a consumer of data supplied by a data provider. By way of example, and not limitation, the term "user" or "subscriber" can refer to a person who receives data provided by the data or service provider over the Internet in a browser session, or can refer to an automated software application which receives the data and stores or processes the data. Those skilled in the art will recognize that the methods and systems of the present disclosure may be implemented in many manners and as such are not to be limited by the foregoing exemplary embodiments and examples. In other words, functional elements being performed by single or multiple components, in various combinations of hardware and software or firmware, and individual functions, may be distributed among software applications at either the client level or server level or both. In this regard, any number of the features of the different embodiments described herein may be combined into single or multiple embodiments, and alternate embodiments having fewer than, or more than, all of the features described herein are possible.

Functionality may also be, in whole or in part, distributed among multiple components, in manners now known or to become known. Thus, myriad software/hardware/firmware combinations are possible in achieving the functions, features, interfaces and preferences described herein. Moreover, the scope of the present disclosure covers conventionally known manners for carrying out the described features and functions and interfaces, as well as those variations and modifications that may be made to the hardware or software or firmware components described herein as would be understood by those skilled in the art now and hereafter.

Furthermore, the embodiments of methods presented and described as flowcharts in this disclosure are provided by way of example in order to provide a more complete understanding of the technology. The disclosed methods are not limited to the operations and logical flow presented herein. Alternative embodiments are contemplated in which the order of the various operations is altered and in which sub-operations described as being part of a larger operation are performed independently.

While various embodiments have been described for purposes of this disclosure, such embodiments should not be deemed to limit the teaching of this disclosure to those embodiments. Various changes and modifications may be made to the elements and operations described above to obtain a result that remains within the scope of the systems and processes described in this disclosure.

Further aspects of the present application are set out in the following numbered paragraphs (NPs):
NP1. A method comprising:
   identifying sensor data collected by a sensor at a location for a time period, the sensor data related to atmospheric conditions proximate to an asset at the location during the time period;
   analyzing the sensor data, and determining attributes of location conditions related to the asset;
   compiling, based on the determined attributes of the location conditions, a vertical column data structure, the vertical column data structure comprising layered atmospheric data extending upwards from a surface level of the asset to a predetermined value above the surface level; and
   storing, over a network, a layered form of data for the asset based at least on the vertical column data structure in a database.
NP2. The method of NP1, further comprising:
   identifying information related to another sensor for another asset;
   identifying, based on the information for the other sensor, stored layered data for the asset;
   analyzing the information for the other sensor based on the stored layered data; and
   determining a configuration for the other sensor, the configuration comprising a modification to functionality of the other sensor that causes additional capabilities to be present on the other sensor.
NP3. The method of NP2, further comprising:
   configuring the other sensor based on the determined configuration; and
   operating the configured other sensor in relation to the other asset, the operation comprising the additional capabilities.
NP4. The method of NP3, wherein a vertical column data structure and another layered form of data are generated from the operation of the configured sensor.
NP5. The method of NP1, further comprising:
   receiving a request for data about an event;
   identifying, from the database, event information related to the asset, wherein the event information comprises the layered data stored in the database for asset; and
   generating, based on the event information, a renderable simulation that provides a visual depiction of atmospheric conditions in the vertical column above the asset in relation to a time of the event.
NP6. The method of NP5, further comprising:
   causing a rendering of the renderable simulation on a device associated with a user that provided the request.
NP7. The method of NP5, wherein the event corresponds to at least one of an occurrence of an activity and a non-occurrence of an activity.
NP8. The method of NP1, wherein the database is a blockchain.
NP9. The method of NP1, wherein the asset is an operational item at the location, wherein the item provides a faculty for operators at the location.
NP10. A system comprising:
   a processor configured to:
   identify sensor data collected by a sensor at a location for a time period, the sensor data related to atmospheric conditions proximate to an asset at the location during the time period;
   analyze the sensor data, and determine attributes of location conditions related to the asset;
   compile, based on the determined attributes of the location conditions, a vertical column data structure, the vertical column data structure comprising layered atmospheric data extending upwards from a surface level of the asset to a predetermined value above the surface level; and
   store, over a network, a layered form of data for the asset based at least on the vertical column data structure in a database.
NP11. The system of NP10, wherein the processor is further configured to:
   identify information related to another sensor for another asset;
   identify, based on the information for the other sensor, stored layered data for the asset;
   analyze the information for the other sensor based on the stored layered data; and
   determine a configuration for the other sensor, the configuration comprising a modification to functionality of the other sensor that causes additional capabilities to be present on the other sensor.
NP12. The system of NP11, wherein the processor is further configured to:
   configure the other sensor based on the determined configuration; and
   operate the configured other sensor in relation to the other asset, the operation comprising the additional capabilities.
NP13. The system of NP12, wherein a vertical column data structure and another layered form of data are generated from the operation of the configured sensor.
NP14. The system of NP10, wherein the processor is further configured to:
   receive a request for data about an event;
   identify, from the database, event information related to the asset, wherein the event information comprises the layered data stored in the database for asset; and
   generate, based on the event information, a renderable simulation that provides a visual depiction of atmospheric conditions in the vertical column above the asset in relation to a time of the event.
NP15. The system of NP14, wherein the processor is further configured to:
   cause a rendering of the renderable simulation on a device associated with a user that provided the request.
NP16. The system of NP10, wherein the event corresponds to at least one of an occurrence of an activity and a non-occurrence of an activity.
NP17. A non-transitory computer-readable storage medium tangibly encoded with computer-executable instructions that when executed by a device, perform a method comprising:
   identifying sensor data collected by a sensor at a location for a time period, the sensor data related to atmospheric conditions proximate to an asset at the location during the time period;
   analyzing the sensor data, and determining attributes of location conditions related to the asset;
   compiling, based on the determined attributes of the location conditions, a vertical column data structure, the vertical column data structure comprising layered atmospheric data extending upwards from a surface level of the asset to a predetermined value above the surface level; and
   storing, over a network, a layered form of data for the asset based at least on the vertical column data structure in a database.
NP18. The non-transitory computer-readable storage medium of NP17, further comprising:
   identifying information related to another sensor for another asset;
   identifying, based on the information for the other sensor, stored layered data for the asset;
   analyzing the information for the other sensor based on the stored layered data; and
   determining a configuration for the other sensor, the configuration comprising a modification to functionality of the other sensor that causes additional capabilities to be present on the other sensor.
NP19. The non-transitory computer-readable storage medium of NP18, further comprising:
   configuring the other sensor based on the determined configuration; and
   operating the configured other sensor in relation to the other asset, the operation comprising the additional capabilities, wherein a vertical column data structure and another layered form of data are generated from the operation of the configured sensor.
NP20. The non-transitory computer-readable storage medium of NP17, further comprising:
   receiving a request for data about an event;
   identifying, from the database, event information related to the asset, wherein the event information comprises the layered data stored in the database for asset;
   generating, based on the event information, a renderable simulation that provides a visual depiction of atmospheric conditions in the vertical column above the asset in relation to a time of the event; and
   causing a rendering of the renderable simulation on a device associated with a user that provided the request.

## Claims

1. A method comprising:
identifying sensor data collected by a sensor at a location for a time period, the sensor data related to atmospheric conditions proximate to an asset at the location during the time period;
analyzing the sensor data, and determining attributes of location conditions related to the asset;
compiling, based on the determined attributes of the location conditions, a vertical column data structure, the vertical column data structure comprising layered atmospheric data extending upwards from a surface level of the asset to a predetermined value above the surface level; and
storing, over a network, a layered form of data for the asset based at least on the vertical column data structure in a database.

2. The method of claim 1, further comprising:
identifying information related to another sensor for another asset;
identifying, based on the information for the other sensor, stored layered data for the asset;
analyzing the information for the other sensor based on the stored layered data; and
determining a configuration for the other sensor, the configuration comprising a modification to functionality of the other sensor that causes additional capabilities to be present on the other sensor.

3. The method of claim 2, further comprising:
configuring the other sensor based on the determined configuration; and
operating the configured other sensor in relation to the other asset, the operation comprising the additional capabilities, optionally wherein a vertical column data structure and another layered form of data are generated from the operation of the configured sensor.

4. The method of claim 1, further comprising:
receiving a request for data about an event;
identifying, from the database, event information related to the asset, wherein the event information comprises the layered data stored in the database for asset; and
generating, based on the event information, a renderable simulation that provides a visual depiction of atmospheric conditions in the vertical column above the asset in relation to a time of the event.

5. The method of claim 4, further comprising:
causing a rendering of the renderable simulation on a device associated with a user that provided the request.

6. The method of claim 4, wherein the event corresponds to at least one of an occurrence of an activity and a non-occurrence of an activity.

7. The method of claim 1, wherein the database is a blockchain.

8. The method of claim 1, wherein the asset is an operational item at the location, wherein the item provides a faculty for operators at the location.

9. A system comprising:
a processor configured to:
identify sensor data collected by a sensor at a location for a time period, the sensor data related to atmospheric conditions proximate to an asset at the location during the time period;
analyze the sensor data, and determine attributes of location conditions related to the asset;
compile, based on the determined attributes of the location conditions, a vertical column data structure, the vertical column data structure comprising layered atmospheric data extending upwards from a surface level of the asset to a predetermined value above the surface level; and
store, over a network, a layered form of data for the asset based at least on the vertical column data structure in a database.

10. The system of claim 9, wherein the processor is further configured to:
identify information related to another sensor for another asset;
identify, based on the information for the other sensor, stored layered data for the asset;
analyze the information for the other sensor based on the stored layered data; and
determine a configuration for the other sensor, the configuration comprising a modification to functionality of the other sensor that causes additional capabilities to be present on the other sensor.

11. The system of claim 10, wherein the processor is further configured to:
configure the other sensor based on the determined configuration; and
operate the configured other sensor in relation to the other asset, the operation comprising the additional capabilities, optionally wherein a vertical column data structure and another layered form of data are generated from the operation of the configured sensor.

12. The system of claim 9, wherein the processor is further configured to:
receive a request for data about an event;
identify, from the database, event information related to the asset, wherein the event information comprises the layered data stored in the database for asset; and
generate, based on the event information, a renderable simulation that provides a visual depiction of atmospheric conditions in the vertical column above the asset in relation to a time of the event.

13. The system of claim 12, wherein the processor is further configured to:
cause a rendering of the renderable simulation on a device associated with a user that provided the request.

14. The system of claim 9, wherein the event corresponds to at least one of an occurrence of an activity and a non-occurrence of an activity.

15. A non-transitory computer-readable storage medium tangibly encoded with computer-executable instructions that when executed by a device, perform a method according to any one of claims 1-8.
